**Europäisches Patentamt**

**European Patent Office**

**Office européen des brevets**

(19)

(11) Veröffentlichungsnummer: **0 369 228 B1**

# EUROPÄISCHE PATENTSCHRIFT

(12)

(45) Veröffentlichungstag der Patentschrift:
**18.12.91 Patentblatt 91/51**

(51) Int. Cl.$^5$: **A61K 31/19, A61K 9/46**

(21) Anmeldenummer: **89120046.1**

(22) Anmeldetag: **28.10.89**

(54) **Ibuprofen-Brausezubereitungen.**

(30) Priorität: **12.11.88 DE 3838431**

(43) Veröffentlichungstag der Anmeldung:
**23.05.90 Patentblatt 90/21**

(45) Bekanntmachung des Hinweises auf die
Patenterteilung:
**18.12.91 Patentblatt 91/51**

(84) Benannte Vertragsstaaten:
**AT BE CH DE ES FR GB GR IT LI LU NL SE**

(56) Entgegenhaltungen:
**EP-A- 0 203 768**
**EP-A- 0 228 164**

(73) Patentinhaber: **BAYER AG**
**W-5090 Leverkusen 1 Bayerwerk (DE)**

(72) Erfinder: **Fritsch, Christian, Dr.**
**Schlinghofener Strasse 33**
**W-5068 Odenthal (DE)**
Erfinder: **Gräwingholt, Werner**
**Unterbuschweg 114**
**W-5000 Köln 50 (DE)**

EP 0 369 228 B1

## Beschreibung

Die Erfindung betrifft klar lösende Ibuprofen-Brausezubereitungen sowie Verfahren zu ihrer Herstellung.

Ibuprofen ist ein Arzneistoff mit analgetischer und entzündungshemmender Wirkung, der ähnlich wie Acetylsalicylsäure die Magenschleimhaut reizt und relativ langsam resorbiert wird. Darüber hinaus ist der Geschmack des Ibuprofens so schlecht, daß üblicherweise Tabletten mit einem geschmacksabdeckenden Überzug versehen werden. Da bei der Acetylsalicylsäure die Probleme der Magenschleimhautreizung und der verlangsamten Resorption durch die Anwendung in Form von Brausetabletten und Granulaten zu lösen sind, wurde es in Erwägung gezogen, diese Arzneiform auch für Ibuprofen anzuwenden. Dabei zeigte sich jedoch, daß der Einsatz üblicher Brausegemische nicht zum Erfolg führt.

Trotz vielfältiger Anstrengungen war es nicht möglich, die extrem schwerlöslichen Ibuprofen-Kristalle aus einer Brausezubereitung heraus schnell und vollständig in einem Glas Wasser aufzulösen, so daß stets recht unattraktiv erscheinende Anwendungsformen resultierten.

Die EP-A 228164 beschreibt eine Ibuprofen — bzw. deren Salze — enthaltende Brausezubereitung, welche Ibuprofen nach erfolgter Brausereaktion in suspendierter Form im Glas Wasser enthält. Diese Zubereitung hat den Nachteil, daß die Vorzüge einer Schmerzmittel-Brausetablette, wie bessere Magenverträglichkeit und schneller Wirkungseintritt, von vornherein nicht gegeben sind.

Die DE-A 3638414 beschreibt den Zusatz von Arginin oder Lysin in einer den molaren Anteil übersteigenden Menge, um eine lösliche Form des Ibuprofens zu erhalten. Der Brausesatz enthält als Säurekomponente Natriumhydrogentartrat. Der Zusatz dieser Komponenten bringt große Nachteile mit sich. Arginin und Lysin sind für die Verwendung als pharmazeutischer Hilfsstoff zu teuer und übersteigen die Kosten für den Wirkstoff Ibuprofen. Natriumhydrogentartrat wirkt als Säure so schwach, daß bei einem pH-Wert von über 6,5 für das Gesamtsystem die Intensität der Brausereaktion zu wünschen übrig läßt und fast gar nicht mehr zu erkennen ist. Somit wird der den Patienten positiv ansprechende Brauseeffekt praktisch nicht erreicht.

Die EP-A 203768 beschreibt eine therapeutische Brausezusammensetzung, die Paracetamol, Acetylsalicylsäure oder Ibuprofen enthalten kann. Diese Erfindung schlägt vor, den Wirkstoff (z.B. Ibuprofen) mit einem Granulationshilfsmittel (z.B. PVP) zu granulieren, dieses Granulat mit einem Teil einer Komponente des Brausegemisches zu vermischen und anschließend diese Vormischung mit einem Brausesystem zu mischen. Die beschriebene Vorgehensweise eignet sich zur Herstellung klar löslicher Brausezubereitungen des Paracetamols und des Aspirins, nicht jedoch des Ibuprofens. Entsprechend dieser Erfindung können keine 200 mg Ibuprofen enthaltende Brausezubereitungen hergestellt werden, die sich in 100 bis 200 ml Wasser von 15 bis 25°C so schnell und klar auflösen, daß nach 2 Minuten mehr als 95% des Wirkstoffes gelöst vorliegen.

Bei der Herstellung von Ibuprofen-Brausezubereitungen nach herkömmlichen Verfahren besteht die prinzipielle Schwierigkeit darin, daß bei einem pH-Wert der fertig gelösten Zubereitung von 6,8 (7,2), welcher nötig ist, um 200 (400) mg Ibuprofen in Lösung zu halten, die Brausereaktion zwischen der Carbonat-Komponente und der Säure-Komponente zum Erliegen kommt.

Wird Ibuprofen in der Säureform eingesetzt, so ist die Auflösegeschwindigkeit gering, so daß noch lange ungelöste Rückstände im Glas Wasser zu finden sind. Die Lösungsgeschwindigkeit läßt sich erhöhen, wenn das Natrium-, Kalium- oder Ammoniumsalz des Ibuprofens eingesetzt wird. Jedoch kommt es dann durch den direkten Kontakt von gelöstem Ibuprofen-Salz mit der Säure-Komponente der Brausemischung zu Ausfällungen, welche nur sehr langsam wieder in Lösung gehen.

Es wurde nun eine Brausezubereitung gefunden, welche alle vorher erwähnten Nachteile beseitigt und sich zur Herstellung schnell und klar löslicher Ibuprofen-Brausepräparate eignet. Die erfindungsgemäße Brausezubereitung hat folgende Zusammensetzung :

a) Basisches Granulat bestehend aus

1 Gew.-Teil wasserlösliches Ibuprofensalz,

2 bis 10 Gew.-Teile, bevorzugt 4 bis 7 Gew.-Teile Trägerstoff,

0,3 bis 0,8 Gew.-Teile, bevorzugt 0,4 bis 0,7 Gew.-Teile Stabilisator und

0,2 bis 1 Gew.-Teil, bevorzugt 0,4 bis 0,7 Gew.-Teile Natrium- oder Kaliumcarbonat.

b) Säurekomponente

1 bis 4 Gew.-Teile, bevorzugt 1,5 bis 2,5 Gew.-Teile, bezogen auf 1 Gew.-Teil wasserlösliches Ibuprofensalz.

Als wasserlösliche Ibuprofensalze werden bevorzugt Natrium-, Kalium- und Ammoniumsalze des Ibuprofens eingesetzt, Besonders bevorzugt sind das Natrium- und Kaliumsalz des Ibuprofens.

Geeignet sind auch wasserlösliche Salze vom Ibuprofen mit Aminosäuren, wie z.B. Arginin, Lysin oder Ornithin oder mit anderen pharmazeutisch akzeptablen organischen Aminoverbindungen wie z.B. N-Methylglucosamin, Piperazin, N-(2-Hydroxyethyl)-piperazin oder Tris(Hydroxymethyl)aminomethan.

Die wasserlöslichen Ibuprofen-Salze können Ibuprofen als Racemat (R(−)-Form und S(+)-Form) aber auch lediglich in Form des reinen S(+)-Enantiomeren oder nur in der Form des reinen R(–)-Enantiomeren enthalten.

Die erfindungsgemäße Brausezubereitung bietet eine Lösung für die genannten Probleme.

Überraschenderweise wurde gefunden, daß sich eine klar lösende, deutlich brausende Ibuprofen-Zubereitung herstellen läßt, wenn Ibuprofen in Form seiner wasserlöslichen Salze, vorzugsweise Natrium- oder Kaliumsalze, zusammen mit dem Trägerstoff und dem Stabilisator granuliert wird, und das Granulat anschließend mit einer 5- bis 20-prozentigen, bevorzugt 15- bis 18-prozentigen Lösung des Carbonates besprüht und dann getrocknet wird. Zu diesem basischen Granulat wird eine geeignete Menge der Säurekomponente zugemischt.

Besonders bevorzugt zur Herstellung der Ibuprofen-Brausezubereitung ist das Natriumsalz des Ibuprofens.

Aus der erfindungsgemäßen Zubereitung, bestehend aus basischem Granulat und Säurekomponente können Pulver, Granulate oder Tabletten hergestellt werden. Bevorzugt seien Granulate und Tabletten genannt.

Erfindungsgemäß kann der Trägerstoff eine wasserlösliche, nicht brausende Komponente sein, wie z.B. Saccharose, Lactose, Mannitol, Mononatriumcitrat und Trinatriumcitrat oder eine brausende Komponente wie z.B. Natrium- oder Kaliumhydrogencarbonat, Natriumcarbonat oder Kaliumcarbonat oder Mischungen dieser Komponenten. Vorzugsweise wird Natriumhydrogencarbonat eingesetzt.

Dem Stabilisator kommt in der Erfindung die Bedeutung zu, einmal gelöstes Ibuprofen in Lösung zu halten und Ausfällungen zu verhindern. Für diesen Zweck sind wasserlösliche Polymere wie z.B. Polyvinylalkohol, Polyvinylpyrrolidon, Polyvinylpyrrolidon-Polyvinylacetat-Copolymerisat, Polyethylenglycol, Polyethylenglycol-Polypropylenglykol-Copolymerisat, bevorzugt Polyvinylpyrrolidon, Polyvinylpyrrolidon-Polyvinylacetat-Copolymerisat und Celluloseether, bevorzugt Hydroxypropylmethylcellulose geeignet. Besonders bevorzugt wird Polyvinylpyrrolidon als Stabilisator eingesetzt.

Als Säurekomponente können die für Brausezubereitungen üblichen organischen Säuren eingesetzt werden, wie beispielsweise Citronensäure, Weinsäure, Bernsteinsäure, Maleinsäure, Äpfelsäure, Malonsäure, Adipinsäure, Fumarsäure, Ascorbinsäure, Mononatriumcitrat, Dinatriumcitrat, Kaliumhydrogentartrat oder Natriumhydrogenphosphat. Bevorzugt werden Citronensäure oder Weinsäure eingesetzt.

Die Brausezubereitung kann weitere Zuschlagsstoffe wie Bindemittel, z.B. Glykokoll, Süßungsmittel, z.B. Saccharin oder Cyclamat, Aromastoffe, Netzmittel, z.B. Dioctylnatriumsulfosuccinat oder Natriumlaurylsulfat und Antischaummittel, z.B. Siliconöl enthalten.

Für die Herstellung von Brausetabletten aus dem Brausegranulat ist der Zusatz eines Schmiermittels nötig.

Fehlt dieses Schmiermittel in der Zusammensetzung und belegen sich die Tablettierwerkzeuge mit Pulverbestandteilen, zeigen die erhaltenen Tabletten eine rauhe, unansehnliche Oberfläche. Die Auswahl eines für Brausetabletten geeigneten Schmiermittels gestaltet sich im allgemeinen als außerordentlich schwierig.

Wasserunlösliche Schmiermittel wie z.B. Magnesiumstearat, Stearinsäure, Talkum, Paraffin und hydriertes Rizinusöl trüben die als klar erwünschte Lösung. Als mehr oder weniger gut wasserlösliche, jedoch meist nicht sehr effektive Schmiermittel kommen Polyethylenglykol, Fumarsäure, Adipinsäure, Natriumbenzoat und Natriumstearylfumarat in Frage.

Polyethylenglycol besitzt nur ungenügende Schmiereigenschaften und verlängert die Auflösungszeigen von Brausetabletten beträchtlich. Darüber hinaus mindert die Substanz die innere Festigkeit der Tabletten.

Fumarsäure und Adipinsäure müssen in relativ hoher Konzentration (10 bis 15%) zugesetzt werden. Sie fällen in Ibuprofen-Brausetabletten, bedingt durch ihren sauren Charakter, den Wirkstoff in seiner schwerlöslichen Säureform aus, wodurch unansehnliche Trübungen entstehen. Gleichzeitig mindern die beiden Schmiermittel die Härte der Tabletten.

Natriumstearylfumarat ist in Wasser nur zu ca. 1% löslich. Daher können beispielsweise in einer 3 g schweren Brausetablette nicht mehr als 3% dieser Substanz eingesetzt werden. In dieser Menge reicht die Wirkung als Schmiermittel häufig nicht aus.

Natriumbenzoat muß als wirksamer Hilfsstoff deklariert werden und ist insofern nicht geeignet.

Es wurde gefunden, daß pharmazeutisch akzeptable Salze der Fumarsäure und Adipinsäure, wie die Natrium-, Kalium-, Ammonium-, Calcium- und Magnesiumsalze, bevorzugt die Natrium- oder Kaliumsalze, in einer Konzentration von 3 bis 10%, bevorzugt 5 bis 8%, bezogen auf die gesamte Zubereitung, als Schmiermittel zur Herstellung von Ibuprofen-Brausetabletten besonders geeignet sind. Diese Schmiermittel werden im allgemeinen als feine Pulver mit einer Partikelgröße kleiner als 50, bevorzugt kleiner als 20 Mikrometer der Brausezubereitung, bestehend aus basischem Granulat und Säurekomponente zugemischt. Ebenso ist es möglich, die Schmiermittel aus einer wäßrigen Lösung auf das basische Granulat aufzusprühen und dann die Säurekomponente zuzumischen.

Besonders bevorzugt ist Dinatriumfumarat als Schmiermittel.

Die Erfindung soll durch folgende Beispiele erläutert werden.

Beispiel 1

Ein Ibuprofen-Brausegranulat, mit welchem 200 mg Ibuprofen zu applizieren sind, ist wie folgt zusammengesetzt:

| | |
|---|---|
| Ibuprofen-Natriumsalz | 221,3 mg |
| Polyvinylpyrrolidon | 120,0 mg |
| Natriumhydrogencarbonat | 1 363,7 mg |
| Natriumcarbonat | 100,0 mg |
| Citronensäure | 395,0 mg |

In den Behälter eines Wirbelschichtgranulators werden 136,37 kg Natriumhydrogencarbonat eingewogen. In einem Rührgefäß werden 22,13 kg Ibuprofen-, Natriumsalz, 12,0 kg Polyvinylpyrrolidon und 80 kg Wasser eingewogen und solange gerührt, bis eine klare Lösung entsteht. Die Lösung wird bei einer Heißlufttemperatur von etwa 100°C auf das Natriumhydrogencarbonat aufgesprüht, wobei ein Granulat entsteht. Auf dieses Granulat wird anschließend eine Lösung von 10 kg Natriumcarbonat in 50 kg Wasser gesprüht. Das so mit einer Schicht von Natriumcarbonat überzogene basische Granulat wird anschließend getrocknet.

Zu dem basischen Granulat werden 39,5 kg Citronensäure gemischt, um so das fertige Ibuprofen-Brausegranulat zu erhalten.

Die Konfektionierung kann in Aluminium-Beuteln in Einmalmengen von 2,2 g geschehen. Das in einem Beutel enthaltene Granulat ergibt dann aufgelöst in 100 ml Wasser nach einer Minute eine klare Ibuprofen-Lösung mit einem pH-Wert von 6,7.

Beispiel 2

Es wird entsprechend der Herstellungsweise wie in Beispiel 1 ein basisches Granulat mit folgender Zusammensetzung hergestellt:

| | |
|---|---|
| Ibuprofen-Natriumsalz | 22,13 mg |
| Polyvinylpyrrolidon | 8,00 mg |
| Natriumhydrogencarbonat | 135,00 mg |
| Natriumcarbonat | 15,00 mg |

180,13 kg dieses Granulates werden mit einer Lösung von 15 kg Dinatriumfumarat in 100 kg Wasser in einer Wirbelschichtapparatur besprüht und anschließend getrocknet. Das so vorbehandelte Granulat wird mit 54,87 kg Citronensäure vermischt und anschließend zu Brausetabletten mit einem Einzelgewicht von 2,5 g verpreßt. Die Tabletten haben eine Auflösungszeit von 2 Minuten in 100 ml Wasser und ergeben dabei eine klare Lösung mit einem pH-Wert von 6,8.

**Patentansprüche**

**Patentansprüche für folgende Vertragsstaaten : AT, BE, CH, DE, FR, GB, IT, LI, LU, NL, SE**

1. Ibuprofen-Brausezubereitung, enthaltend
a) ein basisches Granulat bestehend aus 1 Gew.-Teil wasserlöslichem Ibuprofensalz,
2 bis 10 Gew.-Teile Trägerstoff,
0,3 bis 0,8 Gew.-Teile Stabilisator und
0,1 bis 1 Gew.-Teil Natrium- oder Kaliumcarbonat
und
b) 1 bis 4 Gew.-Teile Säurekomponente.
2. Ibuprofen-Brausezubereitung, enthaltend
a) ein basisches Granulat bestehend aus

4

1 Gew.-Teil wasserlöslichem Ibuprofensalz,

4 bis 7 Gew.-Teile Trägerstoff,

0,4 bis 0,7 Gew.-Teile Stabilisator und

0,4 bis 0,7 Gew.-Teil Natrium- oder Kaliumcarbonat

und

b) 1,5 bis 2,5 Gew.-Teile Säurekomponente.

3. Ibuprofen-Brausezubereitung gemäß Anspruch 1 oder 2, dadurch gekennzeichnet, daß als wasserlösliches Ibuprofensalz das Natrium-, Kalium- oder Ammoniumsalz eingesetzt wird.

4. Ibuprofen-Brausezubereitung gemäß Anspruch 1 bis 3, dadurch gekennzeichnet, daß als Stabilisator ein wasserlösliches Polymeres eingesetzt wird.

5. Ibuprofen-Brausezubereitung gemäß Anspruch 1 bis 4, dadurch gekennzeichnet, daß als Stabilisator Polyvinylpyrrolidon eingesetzt wird.

6. Ibuprofen-Brausezubereitung gemäß Anspruch 1 bis 5, dadurch gekennzeichnet, daß als Säurekomponente Citronen- oder Weinsäure eingesetzt wird.

7. Ibuprofen-Brausezubereitung gemäß Anspruch 1 bis 6 in Form von Tabletten oder Granulaten.

8. Ibuprofen-Brausetabletten gemäß Anspruch 7, dadurch gekennzeichnet, daß sie zusätzlich ein Schmiermittel enthalten.

9. Ibuprofen-Brausetabletten gemäß Anspruch 8, dadurch gekennzeichnet, daß als Schmiermittel Natrium- oder Kaliumsalze der Fumar- oder Adipinsäure eingesetzt werden.

10. Verfahren zur Herstellung von Ibuprofen-Brausezubereitungen gemäß Anspruch 1 bis 6, dadurch gekennzeichnet, daß man das wasserlösliche Ibuprofensalz zusammen mit dem Trägerstoff und dem Stabilisator granuliert, das Granulat anschließend mit der Natrium- oder Kaliumcarbonat-Lösung besprüht, trocknet, und zu diesem basischen Granulat die Säurekomponente zumischt.

**Patentansprüche für folgende Vertragsstaaten : ES, GR**

1. Verfahren zur Herstellung von Ibuprofen-Brausezubereitungen enthaltend

a) ein basisches Granulat bestehend aus

1 Gew.-Teil wasserlöslichem Ibuprofensalz,

2 bis 10 Gew.-Teile Trägerstoff,

0,3 bis 0,8 Gew.-Teile Stabilisator und

0,1 bis 1 Gew.-Teil Natrium oder Kaliumcarbonat

und

b) 1 bis 4 Gew.-Teile Säurekomponente, dadurch gekennzeichnet, daß man das wasserlösliche Ibuprofensalz zusammen mit dem Trägerstoff und dem Stabilisator granuliert, das Granulat anschließend mit der Natrium- oder Kaliumcarbonat-Lösung besprüht, trocknet, und zu diesem basischen Granulat die Säurekomponente zumischt.

2. Verfahren zur Herstellung von Ibuprofen-Brausezubereitungen gemäß Anspruch 1, dadurch gekennzeichnet, daß als wasserlösliches Ibuprofensalz das Natrium-, Kalium- oder Ammoniumsalz eingesetzt wird.

3. Verfahren zur Herstellung von Ibuprofen-Brausezubereitungen gemäß Anspruch 1, dadurch gekennzeichnet, daß als Stabilisator ein wasserlösliches Polymeres eingesetzt wird.

4. Verfahren zur Herstellung von Ibuprofen-Brausezubereitung gemäß Anspruch 1, dadurch gekennzeichnet, daß als Stabilisator ein Polyvinylpyrrolidon eingesetzt wird.

5. Verfahren zur Herstellung von Ibuprofen-Brausezubereitung gemäß Anspruch 1, dadurch gekennzeichnet, daß als Säurekomponente Citronen- oder Weinsäure eingesetzt wird.

6. Verfahren zur Herstellung von Ibuprofen-Brausezubereitung gemäß Anspruch 1, dadurch gekennzeichnet, daß Schmiermittel aus der Gruppe Natrium- oder Kaliumsalze der Fumar- oder Adipinsäure eingesetzt werden.

**Claims**

**Claims for the following Contracting States : AT, BE, CH, DE, FR, GB, IT, LI, LU, NL, SE**

1. Effervescent ibuprofen preparation containing

a) basic granules consisting of

1 part by weight of water-soluble ibuprofen salt,

2 to 10 parts by weight of excipient,

0.3 to 0.8 parts by weight of stabiliser and

0.1 to 1 part by weight of sodium carbonate or potassium carbonate

and

b) 1 to 4 parts by weight of acid component.

2. Effervescent ibuprofen preparation containing

a) basic granules consisting of

1 part by weight of water-soluble ibuprofen salt,

4 to 7 parts by weight of excipient,

0.4 to 0.7 parts by weight of stabiliser and

0.4 to 0.7 parts by weight of sodium carbonate or potassium carbonate

and

b) 1.5 to 2.5 parts by weight of acid component.

3. Effervescent ibuprofen preparation according to Claim 1 or 2, characterised in that the sodium, potassium or ammonium salt is employed as the water-soluble ibuprofen salt.

4. Effervescent ibuprofen preparation according to Claim 1 to 3, characterised in that a water-soluble polymer is employed as the stabiliser.

5. Effervescent ibuprofen preparation according to Claim 1 to 4, characterised in that polyvinylpyrrolidone is employed as the stabiliser.

6. Effervescent ibuprofen preparation according to Claim 1 to 5, characterised in that citric acid or tartaric acid is employed as the acid component.

7. Effervescent ibuprofen preparation according to Claim 1 to 6 in the form of tablets or granules.

8. Effervescent ibuprofen tablets according to Claim 7, characterised in that they additionally contain a lubricant.

9. Effervescent ibuprofen tablets according to Claim 8, characterised in that sodium salts or potassium salts of fumaric acid or adipic acid are employed as the lubricant.

10. Process for the preparation of effervescent ibuprofen preparations according to Claim 1 to 6, characterised in that the water-soluble ibuprofen salt is granulated together with the excipient and the stabiliser, the granules are then sprayed with the sodium carbonate or potassium carbonate solution and dried, and the acid component is admixed to these basic granules.

## Claims for the following Contracting States : ES, GR

1. Process for the preparation of effervescent ibuprofen preparations containing

a) basic granules consisting of

1 part by weight of water-soluble ibuprofen salt,

2 to 10 parts by weight of excipient,

0.3 to 0.8 part by weight of stabiliser and

0.1 to 1 part by weight of sodium carbonate or potassium carbonate

and

b) 1 to 4 parts by weight of acid component characterised in that the water-soluble ibuprofen salt is granulated together with the excipient and the stabiliser, the granules are then sprayed with the sodium carbonate or potassium carbonate solution and dried, and the acid component is admixed to these basic granules.

2. Process for the preparation of effervescent ibuprofen preparations according to Claim 1, characterised in that the sodium, potassium or ammonium salt is employed as the water-soluble ibuprofen salt.

3. Process for the preparation of effervescent ibuprofen preparations according to Claim 1, characterised in that a water-soluble polymer is employed as the stabiliser.

4. Process for the preparation of effervescent ibuprofen preparations according to Claim 1, characterised in that a polyvinylpyrrolidone is employed as the stabiliser.

5. Process for the preparation of effervescent ibuprofen preparations according to Claim 1, characterised in that citric acid or tartaric acid is employed as the acid component.

6. Process for the preparation of effervescent ibuprofen preparations according to Claim 1, characterised in that lubricants from the group comprising sodium salts and potassium salts of fumaric acid or adipic acid are employed.

**Revendications**

**Revendications pour les Etats contractants suivants : AT, BE, CH, DE, FR, GB, IT, LI, LU, NL, SE**

1. Composition effervescente d'Ibuprofen, contenant

a) un granulat basique composé de

1 partie en poids de sel d'Ibuprofen soluble dans l'eau,

2 à 10 parties en poids de substance de support,

0,3 à 0,8 partie en poids de stabilisant et

0,1 à 1 partie en poids de carbonate de sodium ou de carbonate de potassium

et

b) 1 à 4 parties en poids de composant acide.

2. Composition effervescente d'Ibuprofen, contenant

a) un granulat basique composé de

1 partie en poids de sel d'Ibuprofen soluble dans l'eau,

4 à 7 parties en poids de substance de support,

0,4 à 0,7 partie en poids de stabilisant et

0,4 à 7 partie en poids de carbonate de sodium ou de carbonate de potassium

et

b) 1,5 à 2,5 parties en poids de composant acide.

3. Composition effervescente d'Ibuprofen selon la revendication 1 ou 2, caractérisée en ce que l'on emploie, comme sel d'Ibuprofen soluble dans l'eau, un sel de sodium, de potassium ou d'ammonium.

4. Composition effervescente d'Ibuprofen selon les revendications 1 à 3, caractérisée en ce que l'on emploie comme stabilisant un polymère soluble dans l'eau.

5. Composition effervescente d'Ibuprofen selon les revendications 1 à 4, caractérisée en ce que l'on emploie comme stabilisant de la polyvinylpyrrolidone.

6. Composition effervescente d'Ibuprofen selon les revendications 1 à 5, caractérisée en ce que l'on emploie comme composant acide de l'acide citrique ou de l'acide tartrique.

7. Composition effervescente d'Ibuprofen selon les revendications 1 à 6 sous forme de tablettes ou de granulés.

8. Composition effervescente d'Ibuprofen selon les revendications 1 à 7, caractérisée en ce qu'elle contient de plus un lubrifiant.

9. Composition effervescente d'Ibuprofen selon les revendications 1 à 8, caractérisée en ce que l'on emploie comme lubrifiant un sel de sodium ou un sel de potassium de l'acide fumarique ou de l'acide adipique.

10. Procédé pour fabriquer des compositions effervescentes d'Ibuprofen selon les revendications 1 à 6, caractérisé en ce qu'on granule le sel d'Ibuprofen soluble dans l'eau ensemble avec la substance de support et le stabilisant, qu'on asperge ensuite le granulat avec la solution de carbonate de sodium ou de carbonate de potassium, qu'on sèche, et qu'on mélange à ce granulat le composé acide.

**Revendications pour les Etats contractants suivants : ES, GR**

1. Procédé pour fabriquer des compositions effervescentes d'Ibuprofen contenant :

a) un granulat basique composé de :

1 partie en poids de sel d'Ibuprofen soluble dans l'eau,

2 à 10 parties en poids de substance de support,

0,3 à 0,8 partie en poids de stabilisant et

0,1 à 1 partie en poids de carbonate de sodium ou de carbonate de potassium

et

b) 1 à 4 parties en poids de composant acide, caractérisé en ce qu'on granule le sel d'Ibuprofen soluble dans l'eau ensemble avec la substance de support et le stabilisant, qu'on asperge ensuite le granulat avec la solution de carbonate de sodium ou de carbonate de potassium, qu'on sèche et qu'on mélange à ce granulat basique le composant acide.

2. Procédé pour fabriquer des compositions effervescentes d'Ibuprofen selon la revendication 1, caractérisé en ce que l'on emploie, comme sel d'Ibuprofen soluble dans l'eau, un sel de sodium, de potassium ou d'ammonium.

3. Procédé pour fabriquer des compositions effervescentes d'Ibuprofen selon la revendication 1, caractérisé en ce que l'on emploie comme stabilisant un polymère soluble dans l'eau.

4. Procédé pour fabriquer des compositions effervescentes d'Ibuprofen selon la revendication 1, caractérisé en ce que l'on emploie comme stabilisant de la polyvinylpyrrolidone.

5. Procédé pour fabriquer des compositions effervescentes d'Ibuprofen selon la revendication 1, caractérisé en ce que l'on emploie comme composant acide de l'acide citrique ou de l'acide tartrique.

6. Procédé pour fabriquer des compositions effervescentes d'Ibuprofen selon la revendication 1, caractérisé en ce que l'on emploie des lubrifiants du groupe des sels de sodium ou de potassium de l'acide fumarique ou de l'acide adipique.